# EUROPEAN PATENT APPLICATION

(11) **EP 2 327 357 A1**
(43) Date of publication of application: **01.06.2011**
(21) Application number: 09177312.7
(22) Date of filing: 27.11.2009
(51) Int. Cl.: A61B 5/00, A61M 5/172, A61N 1/36, A61N 1/378, H01M 2/34, H01M 10/48, H01M 12/00, H02J 7/00, H04R 25/00, H02J 7/08, H01M 2/02

(54) **Detection of water in electrical devices**

(71) Applicant: DEBIOTECH S.A., 1004 Lausanne (CH)
(72) Inventor: Noth, Andrè, 1004 Lausanne (CH); Neftel, Frédéric, 1005 Lausanne (CH); Proennecke, Stephan, 1004 Lausanne (CH)
(74) Representative: Grosfillier, Philippe

(57) **Abstract**

Electrical device comprising a water detecting means and power reduction means, said power reduction means being arranged in a way as to be activated when water is detected by said water detecting means.

## Description

### Field of invention

The present invention relates to electrical devices using a power source with a need to optimize power consumption. The invention applies in particular to electrical devices that require oxygen to function.

### State of the art

Time life of a battery is one of the most sensitive limitations for on the go medical devices like insulin pumps. This limitation is amplified by the user need of small and light devices.

Zinc-air batteries provide a very attractive power source, due to their large content of energy versus volume. However, this type of battery requires oxygen to deliver its power. If oxygen availability is reduced, for instance when the battery is immersed in water, then battery power is also reduced. A complete power interruption may even occur if the level of oxygen is too low, involving the end-of-life of the battery cell.

Monitoring the status of a battery, for instance in an implantable medical device, is disclosed in US patent application US 2005/0102005.

Prior art devices however do not disclose a way to detect a reduction of oxygen around a battery which requires this type of substance to function.

### General description of the invention

One of the objectives of the present invention is to limit the unnecessary power consumption related to wireless communication. An obvious example is, for a portable device, to reduce the wireless communication function while it is immersed into water (e.g. while the user would be swimming). It is obvious that under such circumstances there is limited or even no need for a portable device to operate and/or communicate wirelessly to a remote control or any other device. In addition, it is most probable that the communication would not be effective under such conditions, so that it makes a lot of sense to limit the energy consumption related to such wireless communication.

Another objective of the present invention is to extend the life time of batteries, in particular metal-air batteries (including but not limited to zinc-batteries), when the device is fully or partially immersed in water.

To this effect the invention relates to an electrical device comprising a water detecting means and power reduction means, said power reduction means being arranged in a way as to be activated when water is detected by said water detecting means.

Preferred embodiments of the invention are defined in the dependent claims.

### Detailed description of the invention

The invention is discussed below in a more detailed way with non-limitative illustrated examples shown in the following figures :

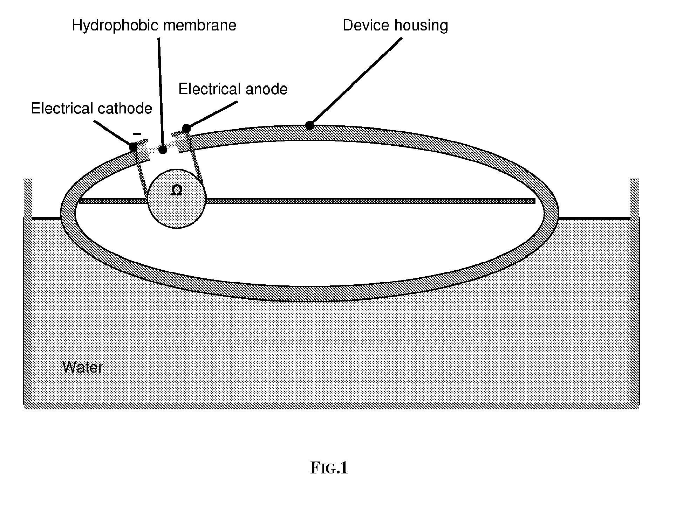

- Figure 1 represents a general view of an embodiment of the invention

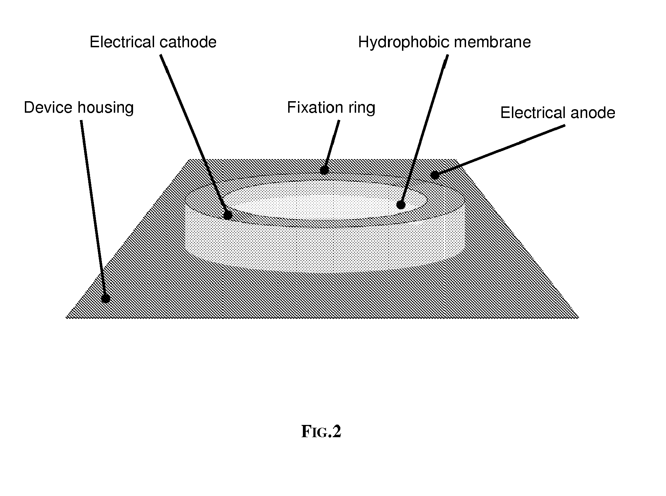

- Figure 2 more precisely illustrates a way to detect water

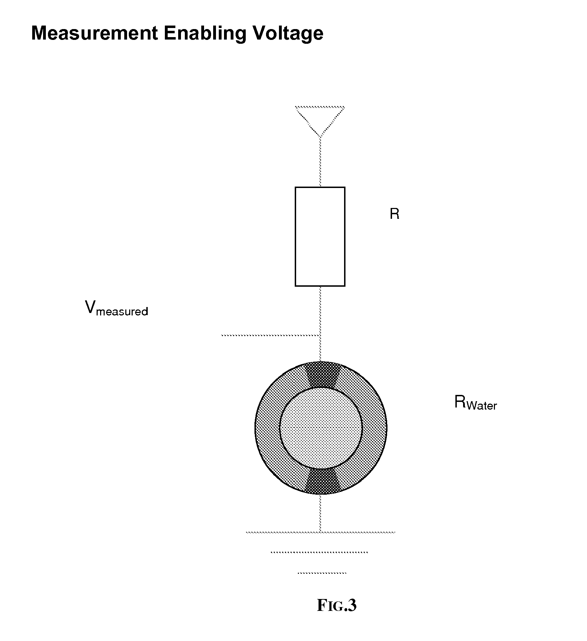

- Figure 3 illustrates one example of electronic circuit for resistance measurement

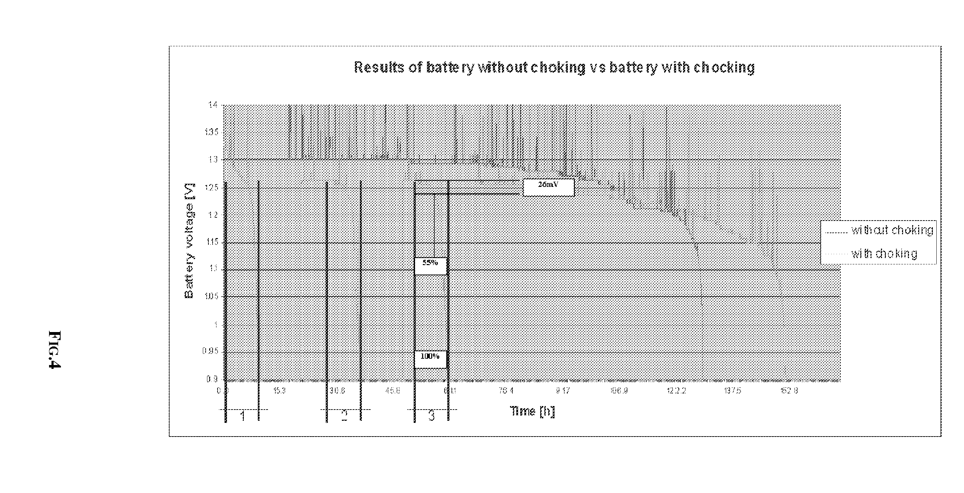

- Figure 4 is a graphical representation of a voltage drop during an anaerobic use of a zinc-air battery.

At least four ways of detecting the presence of water, taken independently or in combination, can be used in the present invention, namely :
- Detection of water on the external surface of the medical device
- Detection of the presence of water through the reduction of oxygen concentration reported by a sensor
- Detection of the presence of water through the reduction of oxygen concentration observed through voltage dropout on the battery (These two points are explained in the document "SKE.D2007 Immersion Detection Study")
- Detection of the presence of water via a wireless radio frequency (RF) link

Water detection must be preferably accurate enough for differencing the fact that the device is surrounded or only sprinkled by water (e.g. difference between a drop of rain and a swimming activity) and/or in a prolonged or brief exposure to water.

The four ways of detecting water are discussed in a more detailed manner below.

### 1.1 EXTERNAL WATER DETECTION

The proposed detection method allows the measurement of the presence of water around the external surface of the device. Two parameters are taken into consideration for the measurement:
- Most favourable location for measuring the presence of water
- Most efficient way to measure the presence of water

In this example these two parameters are defined in accordance with a zinc-air battery, but their definition can be extended to any type of battery cells that requires oxygen.

### 1.1.1 Detection point

The use of a zinc-air battery requires an air inlet. This inlet must prevent the ingress of water, which could alter the electronic. For this reason, a hydrophobic membrane is used. The surface of the membrane represents the last location that will be covered by water and then, is the most favourable location for detecting the presence of water.

### 1.1.2 Detection method

The detection method must take two parameters into account:
- Low power consumption
- Ability to differentiate the fact that the device is immerged into water or only sprinkled.

The most cost effective solution to detect the presence of water is to measure the resistance of a gap between two immerged contact points.

### 1.1.3 Water detection method & equipment

By taking into account the detection method and the best location for detecting water, we propose to implement two electrical contacts on the fixation ring of the air inlet membrane.

The resistance value can be measured using a dedicated circuit. A voltage or potential divider coupled to the contact points on the fixation ring for example offers a range of measurement over several orders of magnitude and is thus very appropriate.

### 1.2 OXYGEN CONCENTRATION REDUCTION THROUGH A DEDICATED SENSOR

The presence of water obstructing the filter can be directly detected through the rarefaction of oxygen inside the device. A dedicated sensor, for example Zirconium dioxide sensors, can offer accurate information about the oxygen concentration.

### 1.3 OXYGEN CONCENTRATION REDUCTION THROUGH THE VOLTAGE DROPOUT OF THE BATTERY

A second way to detect the rarefaction of oxygen is directly monitor the zinc-air battery voltage that decreases. In this case, the battery is used as a sensor, thus requiring no additional component. However, due to the slow initial slope of the voltage drop, there is a significant delay between choking and its detection.

This solution is based on an algorithm implemented in the main processor that compares the actual battery voltage to a predicted battery voltage. The prediction is a function of a history of the pump activities requiring power and a history of previously measurement battery voltages. A pattern corresponding to choke is detected in this data history.

### 1.4 USE OF THE RF LINK

If the device has an RF link with a remote controller, this link can be used for determining when the device is immerged, because the surrounding water decreases the power of the link or even cut it. This effect of surrounding on the RF link is especially efficient for the typical wavelengths that are used for RF transmission, like 2.4 GHz.

The decrease of power of the RF link is measured by emitting after a given while, which could be typically a few minutes, a connection request to a remote control that is associated to the device. When receiving this request, the remote control will acknowledge it with an information of the transmission power of the link. The device can also confirm the measured transmission power when receiving the acknowledge from the remote. The feature for controlling the transmission power of the RF link is available in several standards of RF protocol like Bluetooth.

### 2. COMPLEMENTARY SOLUTIONS

There are at least three complementary solutions to the water detection that can be introduced for mitigating the consequences of a lack of oxygen around the zinc-air battery cells:
- Introduction of a substance being able to degas oxygen when the concentration of oxygen drops down.
- If the device has an RF link with a remote controller, emission of signal to the remote for indicating the fact that water was detected.
- Reduction of the power consumption of the device.

These three solutions will be described more in details in the following sections.

### 2.1 DEGASIFICATION OF OXYGEN

The solution is based on the introduction into the medical device of a substance being able to degas oxygen when the concentration of oxygen drops down. The idea is to use a material which degasses oxygen, for example, when a decrease of the partial pressure of oxygen occurs. However, degassing can also be triggered and controlled by the electronic of the device. For example, the device could heat the degassing substance.

An example of material that could be used is the ceria (or cerium oxide) or combination of metal oxides

The production of oxygen will increase the life time of the battery during immersion of the device.

### 2.2 SIGNAL THE WATER DETECTION TO ITS LINKED REMOTE CONTROL

When water is detected, the medical device can send a signal to its remote control. This signal can be interpreted by the remote control in the following ways:
- As a warning signal indicating that the medical device will be switched into a low-power mode where RF link will be cut. In this case the remote control can display information to the user and wait for the reconnection of the RF link. If the medical device does not reappear after a given time (that can be configured by the user), the remote control could notify to the user that the medical device will stop to work soon due to a loss of power.
- As an indication of water presence around the medical device. Remote control could invite the user to clean her medical device or to protect it from water immersion.

The use of this signal will increase the safety of the medical device.

### 2.3 REDUCTION OF THE POWER CONSUMPTION OF THE DEVICE

The present invention uses at least four different approaches for detecting the presence of water surrounding a device. The presence of this water stops the inhalation of air into a device that needs air for its correct functioning. In particular, this is the case of device that uses zinc-air battery cells. The absence of air will cause a drop of the voltage of the cell, which can kill the battery if it is maintained and/or repeated. For preventing/slowing down this drop, the device can disable or reduce some power consuming activities and limits the function to the essentials, what will request less power. Ultimately, if the battery reaches a predefined minimum threshold, the device can stop all its activities / functionalities. The normal operation starts again only when the inlet of air is reopened and battery voltage is above the minimum threshold. Thus, the ability for detecting the presence of water will allow the device to control its power consumption and adapt it for saving its battery cell in order to avoid killing it.

Alternatively, the reduction of power consumption related to the unnecessary wireless activity while the device is immersed can help increase the power available for the device and increase its usage period.

## Claims

1. Electrical device comprising a water detecting means and power reduction means, said power reduction means being arranged in a way as to be activated when water is detected by said water detecting means.

2. Electrical device according to claim 1 comprising a power source which requires oxygen to function.

3. Electrical device according to claim 2 wherein the power source is a metal-air battery.

4. Electrical device according to claim 3 wherein the metal-air battery is a zinc-air battery.

5. Electrical device according to one of the previous claims wherein said water detecting means are on the external surface of the device.

6. Electrical device according to one of the previous claims wherein said water detecting means comprise an oxygen concentration sensor.

7. Electrical device according to claim 6 wherein said oxygen concentration sensor are based on the measurement of a short term voltage drop-out of the power source.

8. Electrical device according to one of the previous claims 1 to 5 wherein said water detecting means comprise the use of a RF link.

9. Electrical device according to one of the previous claims 1 to 5 wherein said reduction of power consumption relates to the limitation of use of the wireless communication function of such device.

10. Electrical device according to claims 2 to 9 furthermore comprising a signal to be activated when a predefined lack of oxygen is detected. This signal can trigger an alarm or a notice to the user.

11. Electrical device according to one of the claim 10 wherein said signal relates to a remote control device which shall manage certain alarms based on a defined time scale.

12. Electrical device according to claims 2 to 11 furthermore comprising oxygen compensating means adapted to be activated when a predefined lack of oxygen is detected.

13. Electrical device according to claim 12 wherein said oxygen compensating means is a substance being able degas oxygen.

14. Electrical device according to one of the previous claims furthermore comprising power reduction means adapted to be activated when a predefined lack of oxygen is detected.

15. Electrical device according to claim 14 comprising power interrupting means adapted to be activated when a predefined lack of oxygen is detected.

16. Electrical device according to one of the previous claims for use in the medical field.
